(19) Europäisches Patentamt
European Patent Office
Office européen des brevets

(11) **EP 1 176 418 B1**

(12) **EUROPÄISCHE PATENTSCHRIFT**

(45) Veröffentlichungstag und Bekanntmachung des Hinweises auf die Patenterteilung:
**18.05.2016 Patentblatt 2016/20**

(51) Int Cl.:
*G01N 27/414* (2006.01)   *G01N 33/00* (2006.01)

(21) Anmeldenummer: **01106135.5**

(22) Anmeldetag: **13.03.2001**

(54) **Verfahren zur Gasdetektion mit potentialgesteuertem Gassensor**

Method for gas detection with potential controlled gas sensor

Procédé de détection de gaz avec un capteur de gaz commandé par potentiel

(84) Benannte Vertragsstaaten:
**CH DE FR GB IT LI NL**

(30) Priorität: **25.07.2000 DE 10036180**

(43) Veröffentlichungstag der Anmeldung:
**30.01.2002 Patentblatt 2002/05**

(73) Patentinhaber: **Micronas GmbH**
**79108 Freiburg i. Br. (DE)**

(72) Erfinder:
- **Fleischer, Maximilian, Dr.**
  **85635 Höhenkirchen (DE)**
- **Meixner, Hans, Prof.**
  **85540 Haar (DE)**
- **Ostrick, Bernhard**
  **81541 München (DE)**
- **Pohle, Roland, Dr.**
  **85570 Herdweg (DE)**
- **Simon, Elfriede, Dr.**
  **80639 München (DE)**

(74) Vertreter: **Koch Müller**
**Patentanwaltsgesellschaft mbH**
**Maaßstraße 32/1**
**69123 Heidelberg (DE)**

(56) Entgegenhaltungen:
EP-A- 0 947 829     WO-A-00/29829
DE-A1- 3 429 115     DE-A1- 3 526 348
DE-A1- 4 444 607     DE-C1- 3 834 189
US-A- 4 730 479

- OSTRICK B ET AL: "Adsorbed water as key to room temperature gas-sensitive reactions in work function type sensors: the carbonate-carbon dioxide system" SENSORS AND ACTUATORS B, ELSEVIER SEQUOIA S.A., LAUSANNE, CH, Bd. 57, Nr. 1-3, 7. September 1999 (1999-09-07), Seiten 115-119, XP004252993 ISSN: 0925-4005
- OSTRICK B ET AL: "Investigation of the reaction mechanisms in work function type sensors at room temperature by studies of the cross-sensitivity to oxygen and water: the carbonate-carbon dioxide system" SENSORS AND ACTUATORS B, ELSEVIER SEQUOIA S.A., LAUSANNE, CH, Bd. 68, Nr. 1-3, 25. August 2000 (2000-08-25), Seiten 197-202, XP004216614 ISSN: 0925-4005

**Beschreibung**

Die Erfindung betrifft ein Verfahren zur Gasdetektion mit einem potentialgesteuerten Gassensor.

Eine Detektion einer Feuchte ist eine wichtige Anforderungen in der Gassensorik. Dabei ist u. a. eine Anwendung eines kostengünstigen Feuchtesensors gewünscht.

Darüber hinaus zeigen viele Gassensoren eine Feuchte-Querempfindlichkeit, d. h., dass ein Sensorsignal bei einer Detektion eines zu detektierenden Gases ("Zielgas") von der relativen Feuchte rF abhängt. Diese kann einerseits zu einer veränderten Gassensitivität führen, zum anderen gibt es viele Gasreaktionen, die erst in feuchter Umgebung ermöglicht werden, siehe beispielsweise: T. Doll et al., "Ozone Detection in the PPB Range with Work Function Sensors Operating at Room Temperatur", Sensors and Actuators B 34 (1996) pp. 506-510. Zur Bestimmung der Feuchte und gegebenenfalls zur Korrektur des Sensorsignals ist es notwendig, parallel zur Detektion des Zielgases die Feuchte zu messen, siehe dazu: M.G. Buehler M. A. Ryan, "Temperature and Humiditiy Dependence of a Polymer-Based Gas Sensor", SPIE 3082 (1997) pp. 40-48 oder: J. Clements et al., "Novel, Self-Organising Materials for Use in Gas Sensor Arrays: Beating the Humidity Problem", Sensors and Actuators B 47 (1998) pp. 37-42.

Das Problem einer Feuchtemessung bzw. einer Korrektur von Sensordaten tritt insbesondere beim Betrieb eines Gassensors bei einer Temperatur T < 100°C auf. Eine geringe Betriebstemperatur ist aber wünschenswert zur Verringerung einer Leistungsaufnahme oder zur Verwendung temperatursensibler Bauelemente. In die Gruppe der Niedrigtemperatur-Gassensoren gehören beispielsweise Mikrokelvinsonden oder auf Silizium-Technologie basierende Feldeffekttransistoren.

Bisher sind im wesentlichen die folgenden Prinzipien zur Feuchtemessung bekannt:

-   kapazitive Luftfeuchtemessung. Beispielsweise aus: M. Matsuguchi et al. "Characterization of Polymers for a Capacitive-Type Humidity Sensor Based on Water Sorption Behavior", Sensors and Actuators B49 (1998) 179-185 ist dazu eine hygroskopische Polymerschicht bekannt, deren Dielektrizitätskonstante durch Wasseraufnahme entsprechend der relativen Feuchte rF verändert wird. Die dadurch veränderte Kapazität eines Dünnschicht-Kondensators ist direkt proportional zur relativen Feuchte rF.

-   Psychrometrische Luftfeuchtemessung. Bei diesem Prinzip wird mittels eines trockenen und eines befeuchteten Temperaturfühlers aufgrund einer Verdunstung des feuchten Fühlers ein Temperaturunterschied zwischen den beiden Fühlern und daraus eine Luftfeuchte rF ermittelt.

-   Hygrometrische Luftfeuchtemessung. Ein hygrometrischer Messwertgeber ist mit einem Material ausgestattet, welches sich je nach Feuchte dehnt oder zusammenzieht. Durch Messung der Dehnung wird auf die Luftfeuchte rF zurückgeschlossen.

-   Taupunktspiegelhygrometer. Bei diesem Messverfahren wird eine Temperatur einer verspiegelten Fläche so weit abgekühlt, bis diese anfängt, zu beschlagen. Die in diesem Moment gemessene Temperatur entspricht der Taupunkttemperatur.

Jedes dieser Messsysteme ist vergleichsweise kostenintensiv und für viele Anwendungen nicht geeignet, beispielsweise aufgrund eines hohen Platzbedarfs oder einer geringen Erschütterungstoleranz.

Aus: J. Clements et al., "Novel, Self-Organising Materials for Use in Gas Sensor Arrays: Beating the Humidity Problem", Sensors and Actuators B47 (1998) pp. 37-42, ist eine Überlegung bekannt, Schichten zu entwickeln, die nur eine geringe Feuchte-Querempfinglichkeit aufweisen.

Aus: K. Korsah, C.L. Ma, B. Dress, "Harmonic Frequency Analysis of SAW Resonator Chemical Sensors: Application to the Detection of Carbon Dioxide and Humidity", Sensors and Actuators B 50 (1998) 110-116 oder: A. E. Hoyt et al., "Simultaneous Measurement of CO2 and Humidity Using a Pair of SAW Devices and Cluster-Analysis Pattern Recognition", Tagungsband Transducers '97, Chicago 1997, 1339-1342 ist eine Methode bekannt, ein Zielgas und eine Feuchte gleichzeitig mit verschiedenen Schichten zu messen und mittels Mustererkennung oder Frequenzanalyse eine Gasdetektion durchzuführen. Die Auslesung dieser Feuchtesensoren, die meist auf einer organischen Polymerschicht basieren, erfolgt z. B. mittels eines Oberflächenwellenfilters, einer Widerstandsmessung oder einer Messung einer Änderung von Dielektrizitätseigenschaften, siehe dazu: D. Rebiere et. al., "Synthesis and Evaluation of Fluoropolyol Isomers as SAW Microsensor Coatings: Role of Humidity and Temperature", Sensors and Actuators B 49 (1998) pp. 139-145 oder: R. Buchold et. al., "Design Studies on Piezoresistive Humidity Sensors", Sensors and Actuators B53 (1998) pp. 1-7.

Diese Ausleseverfahren sind mit hohen Kosten, insbesondere bezüglich einer Elektronik, verbunden bzw. erfordern eine häufige Rekalibrierung.

Aus der EP 0.947 829 A1 ist ein Gassensor zur Detektion von Kohlendioxid durch Messung der Austrittsarbeit von Carbonaten oder Phosphaten bekannt, bei dem die Gasdetektion durch Messung der Änderung der Austrittsarbeit an der Oberfläche der gassensitiven Schicht erfolgt. Aus der DE 34 29 115 A1 und der DE 35 26 348 A1 sind weitere GasFETs bekannt.

Es ist Aufgabe der vorliegenden Erfindung, eine einfache, universell einsetzbare und kostengünstigen Methode zur Feuchtemessung und / oder Reduzierung der Feuchte-Querempfindlichkeit auch bei einer niedrigen Temperatur bereitzustellen.

Diese Aufgabe wird mittels eines Verfahrens zur Gasdetektion nach Anspruch 1 gelöst.

Dazu wird ein potentialgesteuerter Gassensor verwendet, der mindestens einen gassensitiven Bereich aufweist, welcher unabhängig von einer Feuchte C polarisierbar ist und dessen relative Dielektrizitätskonstante $\varepsilon_r$ von der Feuchte C abhängig ist. Dabei ist die Unabhängigkeit von der Feuchte C so zu verstehen, daß sich Änderungen der Feuchte C nicht signifikant auf die Oberflächenladung $\sigma$ auswirken.

Unter "polarisierbar" wird verstanden, dass der gassensitive Bereich entweder ein Elektret, d. h. ein permanent polarisiertes Material mit annähernd konstanter Oberflächenladung $\sigma$, enthält oder ein Material, dessen Polarisierungsgrad bzw. Oberflächenladung $\sigma$ durch Anlegen eines Steuersignals, beispielsweise einer Spannung, einstellbar ist. Es werden selbstverständlich auch Elektrete gemeint, welche zusätzlich durch Anstellen eines Steuersignals ihren Polarisationsgrad definiert verändern können.

Bei einem potentialgesteuerten Gassensor, wie einem Feldeffekttransistor oder einer Mikro-Kelvinsonde, ergibt sich eine meßbare Potentialänderung $\Delta\Phi$ aufgrund der Anwesenheit der Oberflächenladung $\sigma$ des gassensitiven Bereichs gemäß

$$\Phi = \sigma \cdot d \;/\; (\varepsilon_r(C) \cdot \varepsilon_0), \qquad\qquad [1]$$

wobei d eine Dicke des gassensitiven Bereichs, $\varepsilon_r$ die von der Feuchte C abhängige relative Dielektrizitätskonstante und $\varepsilon_0$ die Dielektrizitätskonstante des Vakuums darstellt. Dies entspricht dem Prinzip einer Messung einer Austrittsarbeit. Eine prinzipielle Funktionsweise eines potentialgesteuerten Sensors bzw. einer Messung der Austrittsarbeit ist beispielsweise in Reedyk und Perlman, "The Measurement of Surface Charge", J. Electrochem. Soc., 15 (1) 1968 pp. 49-51 beschrieben. Typischerweise wird die Potentialänderung $\Delta\Phi$ in Form einer Spannung U bzw. einer Spannungsänderung $\Delta$U gemessen.

Zur Feuchtedetektion wird eine Änderung der relativen Dielektrizitätskonstante $\varepsilon_r$ (C) verwendet. Eine typische Änderung, liegt zwischen $\varepsilon_r$ = 5 (für keramische Materialien) und $\varepsilon_r$ = 80 (für Wasser). Ausgehend von G1. [1] ergibt sich dann eine Potentialänderung $\Delta\Phi$ bei einem Wechsel von einer Feuchte (C0) zu einer anderen Feuchte (C1) gemäß

$$\Delta\Phi = \sigma \cdot d \;/\; \varepsilon_0 \cdot [1/\varepsilon_r(C1) \;-\; 1/\varepsilon_r(C0)]. \qquad\qquad [2]$$

Dieser Gassensor besitzt den Vorteil, dass mittels einer Variation der Oberflächenladung $\sigma$ und/oder, insbesondere bei einem Elektret, durch Variation der Schichtdicke d entweder gezielt eine Feuchteempfindlichkeit eingestellt oder eine Feuchte-Querempfindlichkeit reduziert bzw. eliminiert werden kann.

Zudem ist die Verwendung des polarisierbaren Bereichs preiswert und platzsparend möglich, beispielsweise als Schicht innerhalb eines FETs oder einer Mikro-Kelvinsonde.

Hinzu kommt, dass keine besonderen Anforderungen an mechanische Anforderungen gestellt werden brauchen , beispielsweise gegenüber einer Vibration oder einer hohen Beschleunigung.

Es ist zur variablen Einstellung der Messempfindlichkeit vorteilhaft, wenn die Oberflächenladung $\sigma$ des gassensitiven Bereichs mittels eines Steuersignals einstellbar ist.

Es ist ebenfalls vorteilhaft, wenn der gassensitive Bereich ein Elektret enthält, weil so auf ein Steuersignal verzichtet werden kann. Die Feuchteempfindlichkeit, also der Bereich einer möglichen Potentialänderung $\Delta\Phi$, kann dann gemäß G1. [2] durch eine Wahl der Schichtdicke d dem Bereich eines erwarteten Sensorsignals angepasst werden.

Lässt sich der Polarisationsgrad des Elektrets zusätzlich durch ein Steuersignal ändern, so ist die Messempfindlichkeit auch durch eine Änderung des Steuersignals anpassbar.

Eine einstellbare Feuchteempfindlichkeit, sei es bei Elektreten über die Einstellung der Schichtdicke d oder bei variabel polarisierbaren Materialien über das Steuersignal, kann beispielsweise dazu verwendet werden, um

a) eine Feuchte zu detektieren;

b) eine Feuchte-Querempfindlichkeit durch geeignete Wahl der Schichtdicke d oder des Steuersignals zu eliminieren;

c) die Feuchteempfindlichkeit mittels des polarisierbaren gassensitiven Bereichs so einzustellen, dass die Feuchte-Querempfindlichkeit einer anderen Sensorschicht kompensierbar ist.

Es ist vorteilhaft, wenn der Gassensor mehrere Elektret enthaltende gassensitive Schichten aufweist, wobei jede dieser Schichten eine unterschiedliche Dicke d aufweist. Dadurch kann eine Potentialdifferenz $\Delta\Phi$ gemäß G1. [2], beispielsweise eine Spannungsdifferenz, auf einfache Weise berechnet werden. Zusätzlich kann durch eine geeignete Wahl der Schichtdicken d ein für einen bestimmten Messbereich optimales Messsignal erreicht werden.

Es ist aufgrund der empfindlichen Messung und kleinen Bauteilvolumen vorteilhaft, wenn der Gassensor als Kelvinsonde vorliegt, insbesondere als Mikro-Kelvinsonde.

Es ist auch günstig, wenn der Gassensor als FET vorliegt, insbesondere als GasFET, CCFET ("Capacitively Coupled FET") oder als SGFET ("Suspended Gate FET"). Der Einsatz eines FET führt zu einem kleinen, robusten, einfach, preisgünstig herstellbaren sowie gut zu handhabenden Gassensor.

Es ist ebenfalls günstig, wenn der gassensitive Bereich $BaCO_3$ enthält.

Zur Reduzierung einer Feuchte-Querempfindlichkeit wird es bevorzugt, wenn eine Schichtdicke d und/oder eine Oberflächenladung $\sigma$ so eingestellt wird, dass zumindest annähernd die Gleichung

$$k \cdot T/(n \cdot e) \; [\ln(C1) - \ln(C0)] \approx \sigma \cdot d/\varepsilon_0 \cdot [1/\varepsilon_r(C0) - 1/\varepsilon_r(C1)] \quad [3],$$

gilt, wobei k die Boltzmann-Konstante, T die absolute Temperatur, e die Elementarladung, n einen Zahlenfaktor und C0 bzw. C1 einen Wert einer Feuchte im Zustand 0 bzw. 1 darstellen. In diesem Fall ist es möglich, dass sich die beiden Feuchteeinflüsse im Zustand 0 und 1 zumindest gegenseitig teilweise aufheben.

Es ist insbesondere vorteilhaft, wenn mindestens zwei gassensitive und Elektret-enthaltende Schichten mit jeweils unterschiedlichen Schichtdicke d1 bzw. d2 eingesetzt werden. Weisen diese beiden gasempfindlichen Schichten neben einer spezifischen Sensitivität gegenüber einem Zielgas ebenfalls eine Feuchte-Querempfindlichkeit gemäß G1. [1] auf, so gelten für das Sensorsignal S1 der ersten Schicht und für das Sensorsignal S2 der zweiten Schicht die Gleichungen:

$$S1 = SZ + \sigma \cdot d1 \; / \; \varepsilon_r \varepsilon_0$$
$$S2 = SZ + \sigma \cdot d2 \; / \; \varepsilon_r \varepsilon_0 \qquad\qquad [4].$$

Daraus lässt sich das nur durch das Vorhandensein des Zielgases ausgelöste Sensorsignal SZ

$$SZ = (d2 \; S1 - d1 \; S2) \; / \; (d2 - d1) \qquad\qquad [5]$$

unabhängig von einer Feuchte C sowie das Sensorsignal SF aufgrund der Feuchte aus

$$SF = \sigma \cdot (d1 - d2) \; / \; (\varepsilon_r \cdot \varepsilon_0) \qquad\qquad [6]$$

berechnen. Dies ist eine besonders einfache und effektive Methode zur Reduzierung bzw. Elimination der Feuchte-Querempfindlichkeit.

Es ist auch günstig, wenn der Gassensor mehrere gassensitive Bereiche aufweist, von denen mindestens einer polarisierbar ist. Dann kann eine Feuchte-Querempfindlichkeit f(C0,C1) durch die bekannte Feuchte-Empfindlichkeit der polarisierbaren Schicht gemäß G1. [2],[3] dadurch kompensiert werden, dass dessen Feuchte-Querempfindlichkeit dieser gemäß

$$f(C0,C1) \approx \sigma \cdot d/\varepsilon_0 \cdot [1/\varepsilon_r(C0) - 1/\varepsilon_r(C1)] \qquad\qquad [7]$$

zumindest annähernd gleichgesetzt wird. Diese Kompensation kann beispielsweise mittels einer einfachen Verschaltung hergestellt werden.

Selbstverständlich ist es auch möglich, auf diese Weise eine Feuchte-Querempfindlichkeit eines weiteren, autonomen Gassensors ohne polarisierbare Schicht zu kompensieren, indem dieser weitere Gassensor und ein einen polarisierbaren gassensitiven Bereich aufweisender Sensors extern werden und die Sensorsignale beispielsweise in einer separaten Auswerteeinheit, beispielsweise einem Mikroprozessor, zusammengeführt werden. In den folgenden Ausführungsbeispiele wird ein potentialgesteuerter Gassensor anhand von mehreren Arten von Feldeffekttransistoren ("FETs") mit jeweils einer Elektret-enthaltenden gassensitiven Schicht schematisch näher dargelegt.

Figur 1    zeigt Feuchtesignale bei einer Auftragung einer gemessenen Potentialdifferenz gegen eine relative Feuchte;
Figur 2    zeigt ein GasFET ohne Luftspalt mit potentiometrischer Kopplung;
Figur 3    zeigt ein CCFET ohne Luftspalt mit kapazitiver Kopplung,
Figur 4    zeigt ein SGFET mit Luftspalt und potentiometrischer Kopplung.

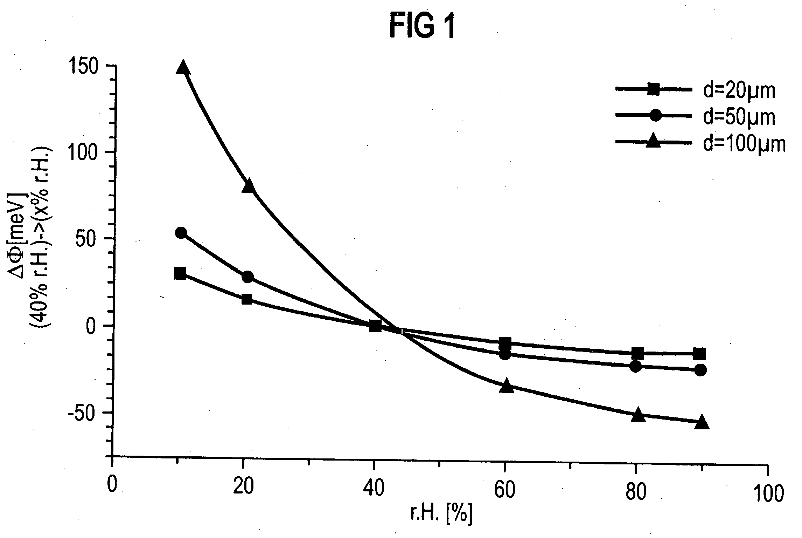

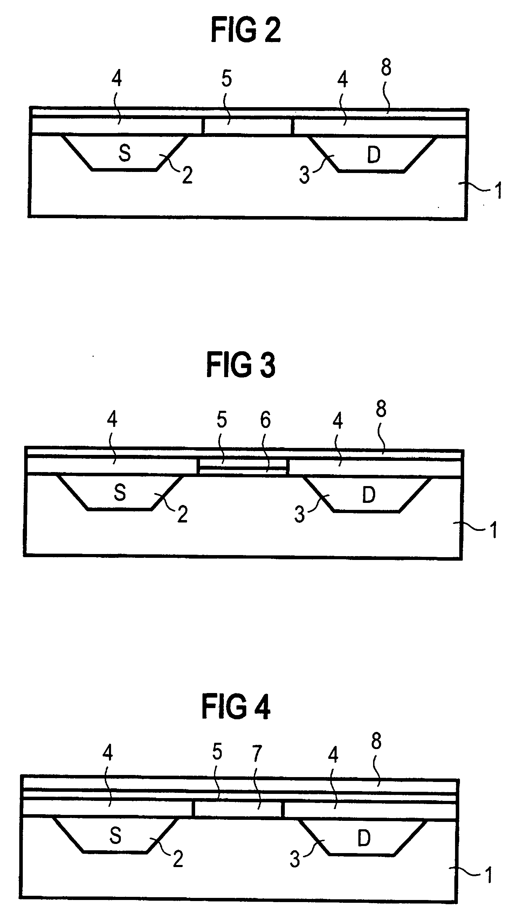

Es sei angemerkt, dass die Ausführungsformen entsprechend der Figuren 2 und 3 nicht beansprucht sind. Figur 1 zeigt eine Auftragung einer Potentialdifferenz $\Delta\Phi$ als Spannungsdifferenz $\Delta U$ in meV gegen eine relative Feuchte rF in Prozent bei Variation einer Schichtdicke d einer aus Elektret bestehenden gassensitiven Schicht.

Vorausgesetzt wird eine Oberflächenladung von $2,0 \times 10^7$ e/cm². Das Sensorsignal $\Delta U$ ist für eine Konzentration relativer Feuchte rF von 40% bei 25°C angegeben. Die relative Dielektrizität $\varepsilon_r$ (C) variiert dabei zwischen 5 und 70. Die Werte sind angegeben für eine Schicht aus $BaCO_3$. $BaCO_3$ ist ein polykristallines Material, bei dem sich die relative Dielektrizitätskonstante $\varepsilon_r$ unter Feuchtebeaufschlagung ändert.

Man erkennt deutlich, dass die Empfindlichkeit des Sensorsignals $\Delta U$ stark von der Dicke d des gassensitiven Bereichs 5, hier als Schicht vorliegend, abhängt. In diesem Fall führt eine größere Schichtdicke d zu einer signifikant höheren Messgenauigkeit.

Figur 2 zeigt als Schnittdarstellung in Seitenansicht den Aufbau eines GasFETs ohne Luftspalt, wobei der Ausdruck "GasFET" für einen allgemeinen gassensitiven Feldeffekttransistor steht.

Dabei sind in ein Basiselement 1 aus Silizium ein Sourcebereich 2 und ein Drainbereich 3 eingebracht, welche mittels einer Passivierungsschicht 4, welche am Kanal geöffnet ist, überdeckt werden. Zwischen Source 2 und Drain 3 ist ein gassensitiver Bereich 5 In Form einer gassensitiven Schicht aus Elektret vorhanden, welches porös und gasdurchlässig ist. Als obere Deckschicht ist eine Metallisierung 8 vorhanden, welche ebenfalls porös und gasdurchlässig ist.

Durch die Metallisierung 8 gelangt Feuchte, also Wassermoleküle, an den gassensitiven Bereich 5, welcher daraufhin den Wert seiner Dielektrizitätskonstante $\varepsilon_r$ (C) ändert. Dadurch ändert sich wiederum das am Feldeffekttransistor abgreifbare Sensorsignal $\Delta\Phi$ bzw. $\Delta U$.

Figur 3 zeigt einen CCFET (Capacitively Coupled FET), bei dem zwischen dem gassensitiven Bereich 5 und dem Basiselement 1 eine elektrisch floatende Metallisierung 6 vorhanden ist.

Figur 4 zeigt einen SGFET (Suspended Gate FET) mit einem Luftspalt 7 bei potentiometrischer Koppelung. Dabei ist zum Einlass des Gases unter dem porösen und gasdurchlässigen Elektret ein Luftspalt vorhanden.

In diesen Ausführungsbeispielen ist es auch möglich, den gassensitiven Bereich 5 nicht mittels eines Elektrets, sondern einer variabel polarisierbaren Schicht auszuführen, so dass der gassensitive Bereich 5 mittels eines Steuersignals, typischerweise einer Spannung, definiert vorpolarisiert wird. Dies ist z. B. günstig für den Fall, dass die Oberflächenladung $\sigma$ auf der Sensorschicht 5 nicht stabil ist, sondern sich zeitlich verändert. Dies würde ohne definiertes Vorpolarisieren zu einer Veränderung der Messempfindlichkeit führen.

Bei Verwendung einer Vorspannung sind prinzipiell alle polarisierbaren Materialien zur Feuchtedetektion geeignet, da sich durch die Vorspannung eine definierte Oberflächenladung $\sigma$ ausbildet. Ebenfalls ist es möglich, ein Elektret zu verwenden, bei dem zusätzlich durch Anlegen eines Steuersignals eine Oberflächenladung $\sigma$ zusätzlich veränderbar ist.

Bei einer Verwendung der Ausführungsformen als Gassensoren mit verminderter Feuchte-Querempfindlichkeit, d. h. dass durch den gassensitiven Bereich nicht nur eine Feuchte rF, sondern auch ein Zielgas detektiert werden soll, können beispielsweise die folgenden Abwandlungen implementiert werden:

Bei einer Gasdetektion bei T < 100°C laufen viele Gasreaktionen unter Beteiligung der Feuchte ab. Dies gilt insbesondere dann, falls im Reaktionsmechanismus als ein Reaktionsschritt eine typische Säure- oder Basen-Bildungsreaktion beteiligt ist, beispielsweise

$$CO_2 + H_2O \rightarrow H_2CO_3$$

$$2NO_2 + H_2O \rightarrow HNO_2 + HNO_3$$

$$NH_3 + H_2O \rightarrow NH_4OH$$

In diesen Fällen ist mit einer Feuchte-Querempfindlichkeit der jeweiligen Gasreaktion in der Größenordnung einer Potentialänderung gemäß G1. [2] zu rechnen. Diese Feuchte-Querempfindlichkeit kann nicht durch Filtern des Gasstroms eliminiert werden, da die Reaktionen eine wichtige Teilreaktion im Reaktionsmechanismus darstellen. Im Falle einer polarisierbaren Schicht 5 kann sie jedoch vermindert werden, indem man die Oberflächenladung $\sigma$ und/oder

die Schichtdicke d im typischen Arbeitsbereich derart anpasst, dass G1. [3] gilt. In diesem Fall heben sich die beiden Feuchteeinflüsse gegenseitig zumindest teilweise auf.

Werden polarisierbare Schichten verwendet, die neben einer spezifischen Zielgas-Sensitivität ebenfalls aufgrund von G1. [1] eine Feuchte-Querempfindlichkeit zeigen, so kann durch den Vergleich zweier Schichten aus demselben Material, aber unterschiedlicher Dicke d und/oder unterschiedlicher Oberflächenladung $\sigma$ die Feuchte-Querempfindlichkeit vermindert werden. Die Sensorsignale S1 und S2 der zwei unterschiedlich dicken bzw. mit einer unterschiedlichen Ladung $\sigma$ versehenen Schichten berechnen sich dann nach G1. [4], woraus sich ein zusammengesetztes Signal SZ bzw. SF gemäß G1. [5] bzw. G1. [6] ergibt. Es lassen sich also dadurch unabhängige Sensorsignale ohne Querempfindlichkeit konstruieren.

Analog zum Aufbau mit verminderter Feuchte-Querempfindlichkeit kann der gleiche Mechanismus auch unter Verwendung von zwei getrennten Gassensoren und einer geeigneten Verschaltung stattfinden. Dies ist beispielsweise sinnvoll, wenn einer der verwendeten Sensoren eine gassensitive Schicht aufweist, die entweder nicht polarisierbar ist, oder zwar variabel polarisierbar ist, aber nicht mit einer bestimmten sinnvollen Vorspannung betrieben werden soll, oder bei der ein Elektret verwendet wird, wobei es aber keinen Spielraum in der Schichtdicke d gibt.

Zeigt dieser Gassensor weiterhin eine unerwünschte Feuchte-Querempfindlichkeit der Form f(C0,C1), so kann mittels eines oben beschriebenen Feuchtesensors ein Aufbau hergestellt werden, für dessen Feuchte-Empfindlichkeit G1. [7] gilt. Dadurch kann die Feuchte-Querempfindlichkeit des weiteren Sensors durch den Feuchtesensor kompensiert oder zumindest vermindert werden.

## Patentansprüche

1. Verfahren zur Gasdetektion, bei dem
ein Gassensor (G), der als Feldeffekttransistor vorliegt, mit mindestens einem gassensitiven Bereich (5) ausgebildet ist, und der Gassensor (G)
ein Basiselement (1) aus Silizium aufweist, wobei in das Basiselement ein Sourcebereich (2) und ein Drainbereich (3) eingebracht sind und zwischen Sourcebereich (2) und Drainbereich (3) der gassensitive Bereich (5) in Form einer gassensitiven Schicht vorhanden ist, wobei
aufgrund einer Änderung einer Feuchte (C) eine relative Dielektrizitätskonstante ($\varepsilon_r$) mindestens eines polarisierbaren gassensitiven Bereichs (5) verändert wird,
**dadurch gekennzeichnet dass,**
der Sourcebereich (2) und der Drainbereich (3) von einer Passivierungsschicht (4), welche am Kanal geöffnet ist, überdeckt werden und die gassensitive Schicht aus einem Elektret ausgeführt ist, welches porös und gasdurchlässig ist und den Kanal, den Sourcebereich (2) und den Drainbereich (3) überdeckt, wobei zwischen dem Kanal und der gassensitiven Schicht ein Luftspalt ausgebildet ist, und eine Metallisierung (8), welche porös und gasdurchlässig ist, als obere Deckschicht vorhanden ist, und

- mittels der Änderung der relativen Dielektrizitätskonstante ($\varepsilon_r$) eine Potentialänderung ($\Delta\Phi$) zur Detektion der Feuchte gemessen wird.

2. Verfahren nach Anspruch 1, bei dem eine Feuchte-Empfindlichkeit durch Anlegen eines Steuersignals in Form einer Spannung, eingestellt wird.

3. Verfahren nach Anspruch 1, bei dem eine Feuchte-Empfindlichkeit mittels der Einstellung einer Schichtdicke (d, d1, d2) mindestens eines ein Elektret enthaltendes gassensitiven Bereichs (5) bestimmt wird.

4. Verfahren nach einem der Ansprüche 2 oder 3, bei dem zur Reduzierung einer Feuchte-Querempfindlichkeit eine Dicke (d, d1, d2) und / oder eine Oberflächenladung ($\sigma$) des gassensitiven Bereichs (5) so eingestellt wird, das zumindest annähernd die Gleichung

$$k \cdot T / (n \cdot e) \ [\ln(C1) - \ln(C0)] \approx \sigma \cdot d / \varepsilon_0 \cdot [1 / \varepsilon_r (C0) - 1 / \varepsilon_r (C1)]$$

gilt, wobei n ein Zahlenfaktor ist, und C0 bzw. C1 einen Weit einer Feuchte um Zustand 0 bzw. 1 darstellen.

5. Verfahren nach Anspruch 1 bei dem
ein weiterer Gassensor verwendet wird, dessen Feuchte-Querempfindlichkeit mittels der Gleichung

$$f(C0,C1) \approx \sigma \cdot d/\varepsilon_0 \cdot [1/\varepsilon_r(C0) - 1/\varepsilon_r(C1)]$$

kompensiert wird.

**6.** Verfahren nach einem der Ansprüche 1 oder 4, bei dem bei einer Verwendung zweier gassensitiver, Elektret enthaltener Schichten (5), die jeweils eine unterschiedliche Schichtdicke (d1, d2) aufweisen, ein Sensorsignal (SZ) eines zu detektierenden Zielgases und / oder ein Sensorsignal (SF) der Feuchte (C) unter Verwendung der Gleichungen

$$SZ = (d2\ S1 - d1\ S2) / (d2 - d1)$$

bzw.

$$SF = \sigma \cdot (d1 - d2) / (\varepsilon_r - \varepsilon_0),$$

mit S1 dem Sensorsignal einer der zwei gassensitiven Bereiche und S2 dem Sensorsignal der anderen der beiden gassensitiven Schichten berechnet wird.

**Claims**

**1.** Method for gas detection, in which
a gas sensor (G), which is present as a field effect transistor, is formed with at least one gas-sensitive region (5), and the gas sensor (G) comprises a base element (1) of silicon, wherein a source region (2) and a drain region (3) are formed in the base element and the gas-sensitive region (5) is present in the form of a gas-sensitive layer between source region (2) and drain region (3), wherein
on the basis of a change in a moisture (C) a relative dielectric constant ($\varepsilon_r$) of at least one polarisable gas-sensitive region (5) is changed,
**characterised in that**
the source region (2) and the drain region (3) are covered by a passivation layer (4), which is open at the channel, and the gas-sensitive layer is constructed from an electret, which is porous and gas-permeable and covers the channel, the source region (2) and the drain region (3), wherein an air gap is formed between the channel and the gas-sensitive layer, and
a metallisation (8), which is porous and gas-permeable, is present as a top cover layer, and
a potential change ($\Delta\Phi$) for detection of the moisture is measured by means of the change in the relative dielectric constant ($\varepsilon_r$).

**2.** Method according to claim 1, in which a moisture sensitivity is set by applying a control signal in the form of a voltage.

**3.** Method according to claim 1, in which a moisture sensitivity is determined by means of the setting a layer thickness (d, d1, d2) of at least one gas-sensitive region (5) containing an electret.

**4.** Method according to one of claims 2 and 2, in which for reducing a moisture cross-sensitivity a thickness (d, d1, d2) and/or a surface charge ($\sigma$) of the gas-sensitive region (5) is set so that the equation

$$k \cdot T / (n \cdot e)\ [\ln(C1) - \ln(C0)] \approx \sigma \cdot d/\varepsilon_0 \cdot [1/\varepsilon_r(C0) - 1/\varepsilon_r(C1)]$$

at least approximately applies, wherein n is a numerical factor and C0 or C1 represents a value of a moisture in the state 0 or 1, respectively.

**5.** Method according to claim 1, in which use is made of a further gas sensor, compensation for the moisture cross-sensitivity of which is provided by the means of the equation

$$f(C0,C1) \approx \sigma \cdot d/\varepsilon_0 \cdot [1/\varepsilon_r(C0) - 1/\varepsilon_r(C1)].$$

**6.** Method according to one of claims 1 and 4, in which in the case of use of two gassensitive layers (5), which contain electrets and which have respectively different layer thicknesses (d1, d2), a sensor signal (SZ) of a target gas to be detected and/or a sensor signal (SF) of the moisture (C) is or are calculated with respective use of the equations

$$SZ = (d2\ S1 - d1\ S2) / (d2 - d1)$$

or

$$SF = \sigma \cdot (d1 - d2) / (\varepsilon_r - \varepsilon_0),$$

wherein S1 is the sensor signal of one of the two gas-sensitive regions and S2 is the sensor signal of the other one of the two gas-sensitive layers.

**Revendications**

**1.** Procédé de détection de gaz, dans lequel un détecteur de gaz (G) qui est constitué par un transistor à effet de champ est formé avec au moins une zone sensible au gaz (5) et le détecteur de gaz (G) comporte un élément de base (1) en silicium, dans lequel une zone source (2) et une zone drain (3) sont introduites dans l'élément de base et la zone sensible au gaz (5) se trouve entre la zone source (2) et la zone drain (3) sous la forme d'une couche sensible au gaz, dans lequel on modifie, en raison d'une variation d'une humidité (C), une constante diélectrique relative ($\varepsilon r$) d'au moins une zone sensible au gaz polarisable (5), **caractérisé en ce que** l'on recouvre la zone source (2) et la zone drain (3) avec une couche de passivation (4), qui est ouverte au niveau du canal et la couche sensible au gaz est réalisée sous la forme d'un électret, qui est poreux et perméable au gaz et recouvre le canal, la zone source (2) et la zone drain (3), dans lequel un espace d'air est formé entre le canal et la couche sensible au gaz et il se trouve comme couche de recouvrement supérieure une métallisation (8) qui est poreuse et perméable au gaz, et on mesure, au moyen de la variation de la constante diélectrique relative ($\varepsilon r$), une variation de potentiel ($\Delta\phi$) pour la détection de l'humidité.

**2.** Procédé selon la revendication 1, dans lequel on règle une sensibilité à l'humidité en appliquant un signal de commande sous la forme d'une tension.

**3.** Procédé selon la revendication 1, dans lequel on détermine une sensibilité à l'humidité au moyen du réglage d'une épaisseur de couche (d, d1, d2) d'au moins une zone sensible au gaz (5) contenant un électret.

**4.** Procédé selon l'une des revendications 2 ou 3, dans lequel, pour la réduction d'une sensibilité transversale à l'humidité, on règle une épaisseur (d, d1, d2) et/ou une charge de surface ($\sigma$) de la zone sensible au gaz (5) de telle manière que l'équation suivante s'applique approximativement

$$k \cdot T/(n \cdot e)[\ln(C1) - \ln(C0) \approx \sigma \cdot d/\varepsilon 0 \cdot [1/\varepsilon r(C0) - 1/\varepsilon r(C1)]$$

dans laquelle n est un facteur numérique et C0 ou C1 représentent une valeur d'une humidité à l'état 0 ou 1.

**5.** Procédé selon la revendication 1, dans lequel on utilise un autre détecteur de gaz, dont la sensibilité transversale à l'humidité est compensée au moyen de l'équation

$$f(C0,C1) \approx \sigma \cdot d/\varepsilon 0 \cdot [1/\varepsilon r(C0) - 1/\varepsilon r(C1)].$$

**6.** Procédé selon l'une des revendications 1 ou 4, dans lequel, lors d'une utilisation de deux couches sensibles au gaz (5) contenant un électret qui présentent respectivement une épaisseur de couche différente (d1, d2), on calcule un signal de détecteur (SZ) d'un gaz cible à détecter et/ou un signal de détecteur (SF) de l'humidité (C) en utilisant les équations

$$SZ = (d2\ S1 - d1\ S2)/(d2 - d1)$$

ou

$$SF = \sigma \cdot (d1 - d2)/(\varepsilon r - \varepsilon 0),$$

où S1 est le signal de détecteur d'une des deux zones sensibles au gaz et S2 est le signal de détecteur de l'autre des deux couches sensibles au gaz.

FIG 1

EP 1 176 418 B1

# FIG 2

# FIG 3

# FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0947829 A1
- DE 3429115 A1
- DE 3526348 A1

**In der Beschreibung aufgeführte Nicht-Patentliteratur**

- **T. DOLL et al.** Ozone Detection in the PPB Range with Work Function Sensors Operating at Room Temperatur. *Sensors and Actuators,* 1996, vol. B 34, 506-510
- **M.G. BUEHLER ; M. A. RYAN.** Temperature and Humiditiy Dependence of a Polymer-Based Gas Sensor. *SPIE,* 1997, vol. 3082, 40-48
- **J. CLEMENTS et al.** Novel, Self-Organising Materials for Use in Gas Sensor Arrays: Beating the Humidity Problem. *Sensors and Actuators,* 1998, vol. B 47, 37-42
- **M. MATSUGUCHI et al.** Characterization of Polymers for a Capacitive-Type Humidity Sensor Based on Water Sorption Behavior. *Sensors and Actuators,* 1998, vol. B49, 179-185
- **J. CLEMENTS et al.** Novel, Self-Organising Materials for Use in Gas Sensor Arrays: Beating the Humidity Problem. *Sensors and Actuators,* 1998, vol. B47, 37-42
- **K. KORSAH ; C.L. MA ; B. DRESS.** Harmonic Frequency Analysis of SAW Resonator Chemical Sensors: Application to the Detection of Carbon Dioxide and Humidity. *Sensors and Actuators,* 1998, vol. B 50, 110-116
- **A. E. HOYT et al.** Simultaneous Measurement of CO2 and Humidity Using a Pair of SAW Devices and Cluster-Analysis Pattern Recognition. *Tagungsband Transducers,* 1997, vol. 97, 1339-1342
- **D. REBIERE.** Synthesis and Evaluation of Fluoropolyol Isomers as SAW Microsensor Coatings: Role of Humidity and Temperature. *Sensors and Actuators,* 1998, vol. B 49, 139-145
- **R. BUCHOLD.** Design Studies on Piezoresistive Humidity Sensors. *Sensors and Actuators,* 1998, vol. B53, 1-7
- **REEDYK ; PERLMAN.** The Measurement of Surface Charge. *J. Electrochem. Soc.,* 1968, vol. 15 (1), 49-51